# EUROPEAN PATENT APPLICATION

(11) **EP 3 875 074 A1**
(43) Date of publication of application: **08.09.2021**
(21) Application number: 20161201.7
(22) Date of filing: 05.03.2020
(51) Int. Cl.: A61K 8/891, A61Q 19/00, A61K 35/17, A61K 35/19, A61P 17/02, A61K 35/14, C12N 5/00

(54) **BLOOD PREPARATION USEFUL FOR WOUND HEALING AND TISSUE REGENERATION**

(71) Applicant: Novystem S.p.A., 20129 Milano (IT)
(72) Inventor: ERRATICO, Silvia, 20129 Milano (IT); ORLANDI, Massimo, 20129 Milano (IT)
(74) Representative: Bianchetti & Minoja SRL

(57) **Abstract**

The invention provides a blood preparation consisting of platelet- and lymphocyte-enriched plasma, the use thereof for wound healing and body tissue regeneration, pharmaceutical and cosmetic compositions containing it and a method for its preparation.

## Description

The present invention is directed to a blood preparation which contains plasma enriched in platelets and lymphocytes, the use thereof for wound healing and body tissue regeneration, a pharmaceutical composition containing it and a method for its preparation.

### Background of the invention

Skin wound healing is a highly organized and dynamic process played by soluble mediators, blood cells and extracellular matrix that result in the restoration of tissue integrity and functions. Three are the steps required to complete the process: the inflammation phase, characterized by extravasation of blood cells, interleukin chemotaxis and ROS production; the proliferation phase, described by angiogenesis, granulation tissue formation, fibroblast and keratinocytes proliferation, that allows wound closure and re-connection of epidermal and dermal compartments; tissue remodeling, where wound contraction and extracellular matrix reorganization complete tissue repair. An interruption in the normal wound healing process can lead to the development of non-healing chronic wounds, a typical complication of several diseases, such as foot ulcer from diabetes and pressure ulcer resulting from spinal cord injuries. These chronic wounds are a high-prevalence problem in our society, where they represent a great cost to the healthcare system as they heal poorly and recur frequently. In addition to the debridement step, an important component of wound care, different approaches have been proposed to treat these injuries: pharmacological strategies, such as growth factors application or stimulation, different types of wound dressing, and skin substitutes. If the application of simple dressing is not enough, combining the anti-microbial action with the wound closure promotion represents an alternative approach. For extensive wounds, a variety of skin substitutes are available, that can be classified by origin (allogenic, xenogeneic, and autologous), composition (dermal, epidermal or both components) or timing (durable or temporary substitutes). The ideal skin substitute performs the functions of skin, while being cost-effective, widely available, and easy to apply. Another approach proposed, in combination with other treatments, is the topical negative pressure therapy, with effects on angiogenesis, reduction of edema and wound size. In all cases (except the use of autologous skin substitutes), the problem of introducing an external agent in the lesion site could induce an additional inflammatory response in a microenvironment already instable. In literature, PRP (platelet rich plasma) has been reported to be a regenerative treatment successfully used in orthopedic trauma (Sanchez, Anitua et al. 2007, Arora, Ramanayake et al. 2009, Sanchez, Anitua et al. 2009). The use of platelets derivatives represents a way to deliver concentrate growth factors normally released by platelets in the hemostasis process. (Anitua, Andia et al. 2004) This concentration of platelets and application provides a higher amount of bioactive factors that promote wound healing, as reported in different clinical studies (Crovetti, Martinelli et al. 2004, Saldalamacchia, Lapice et al. 2004), but the methodological approach is not always standardized and reproducible. A further improvement in the treatment of ulcers is represented by the activation of PRP adding calcium chloride; this manipulation allows the formation of a fibrin clot, a "gel-like" texture applicable to a tridimensional wound (Anitua, Aguirre et al. 2008, Rainys, Cepas et al. 2019). Together with the extracellular remodeling, impaired wound healing depends also on lack of neovascularization; endothelial progenitor cells (EPCs) are unipotent adult stem cells, derived from haemangioblasts within the bone marrow niche, that were first discovered as circulating cells in peripheral blood. These cells have been isolated by the expression of different markers, such as CD45 to identify hematological origin, in combination with different endothelial surface markers, such as CD31, CD144 or CD146. Additionally, T lymphocytes expressing endothelial potential have been described as Tang (Miao, Qiu et al. 2016, Manetti, Pratesi et al. 2017); angiogenic T cells are commonly identified by the co-expression of CD3, CD31 and CD184. In vitro experiments showed that Tang cells may promote vasculogenesis and endothelial repair by stimulating early EPC through the secretion of high levels of proangiogenic factors, such as vascular endothelial growth factor (VEGF), interleukin (IL)-8, IL-17 and matrix metalloproteinase (MMP)-9 (Hur, Yang et al. 2007).

### Known prior art

EP3403659, in the name of Regenlab, discloses a process for the preparation of a wound healant composition containing a thrombin serum prepared by centrifugation of whole blood in a tube comprising a thixotropic gel. The tissue healant composition contains platelets, white blood cells, fibrinogen, autologous thrombin serum.

EP3111974 discloses a process for the preparation of a cell composition comprising the steps of centrifuging whole blood in a separator tube containing a polyester-based thixotropic gel, separating the enriched platelet plasma from the full plasma and admixing the platelet concentrate with a cell extract, wherein the centrifugation is performed at a force of about 1500 g up to 2000 g in a sufficient length of time to form a barrier between the platelet-containing plasma, the lymphocytes and monocytes and the pellet containing the erythrocytes. The isolated cell composition thus obtained is used for wound or tissue healing and for tissue regeneration and particularly for skin, cartilage, muscle, tendon, adipose tissue, cornea, peripheral nerves, spine or bone regeneration.

EP3395383 and EP2073862 disclose wound healant compositions and methods for preparing them based on centrifugation of whole blood. In one embodiment the whole blood is centrifuged in a separator tube provided with a thixotropic gel and the centrifugation step is performed at a force between about 1500 g up to 1700 g for a time selected from about 3 minutes up to about 15 minutes, preferably at 1500 g for about 8 minutes.

There is a need for a composition suitable for the regeneration of lesioned tissues, particularly skin tissues, which is optimized in terms of cellular components thereby improving the wound healing process, and which can be easily prepared and it is ready to use without the need of complex work procedures in sterile conditions.

### Description of the invention

These objectives are achieved by the blood preparation according to the invention, which consists of platelet- and lymphocyte-enriched plasma (also herein referred to as Angio^{PRP}), which proved particularly effective in regenerating damaged body tissues. The blood preparation was studied *in vitro* on skin cultures and *in vivo* in animal models of skin lesions and it showed marked tissue regenerative and wound healing properties.

The blood preparation was analysed for its cellular composition and it was found to comprise, besides platelets, white blood cells CD45⁺, CD31⁺ and CD34⁻ and red blood cells in specific and reproducible amounts.

Accordingly, in a first embodiment the invention is directed to an isolated blood preparation consisting of platelet- and lymphocyte-enriched plasma for use in the regeneration of body tissues and/or wound healing, containing:
- platelets at a concentration ranging from 110 x 10⁶/ml to 330 x 10⁶/ml, preferably from 150x10⁶/ml to 330 x 10⁶/ml;
- white blood cells CD45⁺, CD31⁺ and CD34⁻ at a concentration ranging from 0.1x10⁶/ml to 2.0x10⁶/ml, preferably from 0.1x10⁶/ml to 0.7x10⁶/ml;
- red blood cells at a concentration ranging from 0 to 0.03x10⁹/ml, preferably less than 0.01x10⁹/ml.

The white blood cells fraction was further analysed for its cellular components and it was found to comprise lymphocytes, monocytes and granulocytes, in the following percent amounts with respect to the total number of white blood cells CD45⁺, CD31⁺ and CD34⁻:
- lymphocytes from 46.20% to 80.60%, preferably from 61.60% to 73.70%;
- monocytes from 7.70% to 40.00%, preferably from 15.90% to 23.10%;
- granulocytes from 3.30% to 32.90%, preferably from 5.90% to 16.70%.

In preferred, independent embodiments of the invention:
- the lymphocytes comprise T cells, angiogenic T lymphocytes (Tang), B cells and NK cells;
- the monocytes are CD14⁺ and CD16⁺ or CD16-
- the granulocytes are CD15⁺/16⁺.

According to another embodiment, the invention provides a method for preparing the lympho-platelet enriched plasma blood preparation, which comprises the following steps:
- centrifuging from 2 to 7ml, preferably from 2 to 4ml of whole blood in a centrifugation tube at 1200-1800 rpm (rounds per minute), preferably at 1500 rpm, for 5 up to 10 min and preferably for 5 minutes at room temperature, thereby obtaining the separation of an upper and lower phase, wherein the upper phase contains the platelet- and lymphocyte-enriched plasma and the lower phase contains red cells;
- collecting the upper phase containing the platelet- and lymphocyte-enriched plasma.

Preferably, the centrifugation tube has a 5 ml inner volume and it is provided with a sept separating the tube in two parts so as to allow the physical separation of the platelet- and lymphocyte-rich plasma phase from red cells. More preferably, the position of the sept can be regulated so as to delimit an upper tube volume from 6 to 7 ml, where the platelet- and lymphocyte-rich plasma phase is located after centrifugation, and a lower tube volume from 3 to 4 ml containing the red cells.

Yet more preferably, the sept consists of a hydrophobic disk barrier or membrane with less than 200 micron porosity and provided with a central hole of 1,5 to 2,5 mm diameter. Suitable materials used in the disk barrier or membrane include, but are not limited to, polyethylene.

In a further aspect, the invention is directed to a blood preparation obtainable by the method herein disclosed, which consists of platelet- and lymphocyte-enriched plasma having the characteristics set out above.

The blood preparation according to the invention is conveniently used for the regeneration of lesioned body tissues and for promoting wound healing. In specific embodiments, the blood preparation as herein defined is used for cosmetic and therapeutic applications, wherein:
- the cosmetic applications include anti-aging, scar repairing, wrinkle, acne, alopecia and burns treatment
- the therapeutic applications include wound healing, treatment of lesions of orthopedic or oncological origin such as bone and cartilage damages, post-surgery lesions or wounds; skin ulcers, decubitus sores, pressure ulcers, venous leg ulcers, arterial ulcers, diabetic foot ulcers, corneal lesions such as scarring of the cornea and corneal ulcers; periodontal tissue damages or periosteal lesions.

The blood preparation of the invention can be used in allogenic or autologous methods of treatment. In a preferred embodiment, the blood preparation is used for autologous applications, i.e. it is administered to the same donor of whole blood from which the lympho-platelet enriched plasma is isolated.

The blood preparation as obtained by the method herein disclosed is ready to use, i.e. it can be applied or administered to subjects in need thereof immediately after its obtainment. Alternatively, it can be frozen and used when needed, or it can be worked e.g. by addition of suitable vehicles, carriers, excipients, preservatives, buffering agents, stabilizers. For example, the blood preparation can be used in gel form by adding calcium chloride solution (2.5 - 10% w/v) directly within the syringe used for its collection.

Accordingly, in a further aspect the invention provides a pharmaceutical or cosmetic composition containing the blood preparation as above defined, together with suitable carriers or excipients.

The invention is further illustrated, without being limited, by the following Figures and Examples.

### Description of the Figures

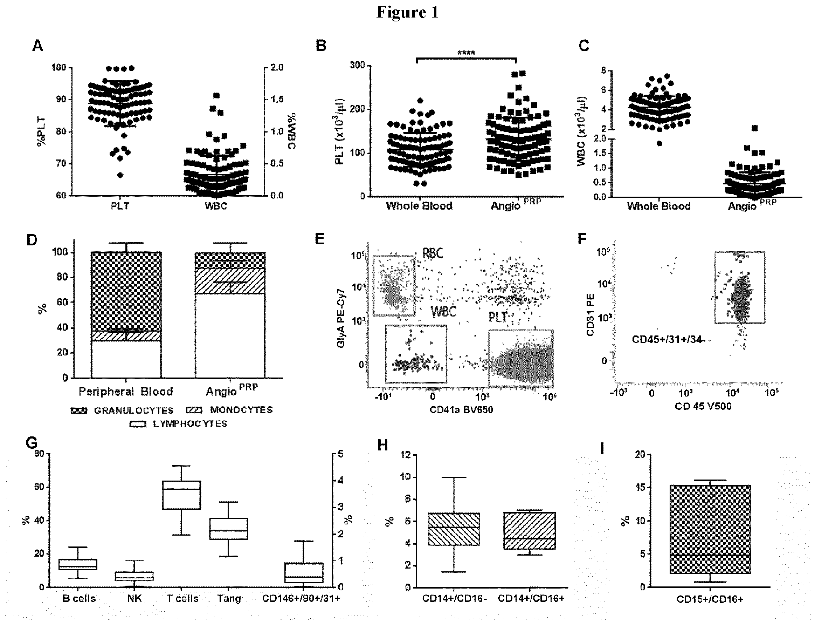

**Figure 1** - **Angio^{PRP} characterization.** Product composition, expressed as percentage of platelets and cells present in Angio^{PRP} (A); analysis of platelets enrichment of Angio^{PRP} compared to original whole blood (B; unpaired t-test, **p<0,01) and comparison of white blood cells concentration before and after separation (C). Graphic representation of Angio^{PRP} cellular subpopulations: granulocytes, monocytes and lymphocytes percentages, compared to whole blood leukocyte formula (D); cytometric analysis of total Angio^{PRP} (E) and cellular compartment, CD45+/CD31+/CD34- for the range 85.00%-97.73% (F). Further cytometric characterization of different cellular subpopulations, expressed as range: lymphocytes (G), monocytes (H) and granulocytes (I).

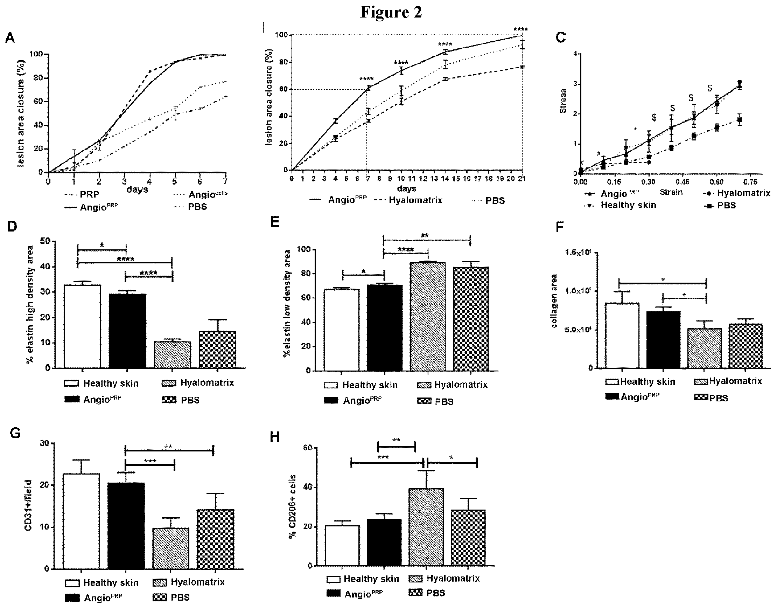

**Figure 2** - **Angio^{PRP} validation in vitro on organotypic culture and in vivo in an animal model.**

Percentage of wound closure area in an in vitro model of skin tissue, treated with Angio^{PRP}, single components (Angio^{cells} and PRP) or PBS as negative control (A). Evaluation of wound closure in vivo in a murine model of skin lesion treated with Angio^{PRP}, Hyalomatrix or PBS. Wound closure rate was quantified as a ratio between the area measured and the area of the initial lesion at different timepoints: 4.7.10.14 and 21 days of treatment (B; ****p<0,0001 two-way analysis of variance (ANOVA) Bonferroni correction). Graphical representation of stress-strain curves obtained from dorsal skin analysis to determine mechanical properties of skin treated with Angio^{PRP} and Hyalomatrix compared to PBS and healthy skin (C, unpaired t-test, *p<0,05). Quantification of high-density (D) and low-density (E) elastin presence (unpaired t-test, *p<0,05; **p<0,01; **** p<0,0001) based on Alcian Blue histological staining of tissues treated with Angio^{PRP} and Hyalomatrix or PBS, compared to healthy skin. Collagen VI area quantification, based on immunofluorescence staining (F, unpaired t-test, *p<0,05). Quantification of angiogenic CD31(G) and inflammatory CD206 (H) positive cells in skin tissue after treatment with Angio^{PRP}, Hyalomatrix or PBS (unpaired t-test, *p<0,05, **p<0.01, ***p<0.001).

### Examples

### Example 1 - preparation of lympho-platelet enriched plasma (Angio^{PRP})

The centrifugation tube used for the separation of the lympho-platelet enriched plasma - hereafter Angio^{PRP} device - is composed of:
1. a main body made by a sterile externally-graduated 5ml polypropylene tube;
2. a rubber closure (BD Vacutainer Haemogard-like);
3. a sept separating the tube in two parts (56% upper volume and 44% lower volume) consisting in a HDPE, porous (pore size<200 micron), hydrophobic, 13 mm diameter and 1.5 mm thickness disk barrier with a central 2 mm diameter hole;
4. a bottom part consisting in a ring nut.

A sample of peripheral blood, previously collected in a sodium citrate tube, is injected through the rubber closure in the tube, the internal barrier retains the blood sample in the upper volume.

The device is then centrifuged at 1500 rpm for 5 minutes. No break deceleration.

The internal membrane facilitates the separation of plasma enriched with platelets (PRP) and cell populations of interest (in the upper part) from waste materials (red blood cells, in the lower part).

The ring nut permits to adjust the whole PRP phase in the upper chamber (above the membrane).

The upper plasma and cells are withdrawn with a syringe and are ready to use in liquid form.

A Live/Dead staining (L3224, Life Technologies, CA, USA) was performed in vitro on Angio^{PRP} after 1, 2, 3, 4 and 7 days in order to evaluate cell survival. Viability decreases during the days, but after 7 days in vitro 82.26±8.50% of cells are alive. For evaluation of endothelial potential, 8x10⁴ HUVEC (ATCC-LGC, VA, USA) were plated on 3D Matrigel (BD Biosciences); 150ul of Angio^{PRP} or PRP or Angio^{cells} was added and after 24 hours cells ramification was quantified by ImageJ software (NIH). Angio^{PRP} induced an amount of ramification longer than other conditions (327,75±9,72x10³ versus 293,05±8,35x10³ µm compared to control condition, t-test *p<0.05).

### Example 2 - FACS analysis

Peripheral blood (PB) was obtained from the blood bank of Department of Transfusion Medicine and Haematology at Policlinico Hospital of Milan, from healthy volunteers after informed consent, according to the guidelines of the Committee on the Use of human Subjects in Research of the Policlinico Hospital of Milan (Milan, Italy). 2.5 ml of peripheral blood, collected in sodium citrate tube were filled into Regeneron device and centrifuged for 5 minutes at 1500 rpm at room temperature in order to induce the phase separation. The platelet-rich-plasma phase and the cells at the interface between red cells and plasma were collected. We analysed pre-separation blood and Angio^{PRP} by blood Coulter counter instrument (DxH 500, Beckman Coulter). Pre-separation blood and cell phase collected were directly labelled with monoclonal antibodies shown below. Cells were incubated with Syto™16, anti-CD45 V500, anti-CD3 V450, anti-CD56 PE-CY7, anti-CD14 APC-H7, anti CD16 PE, anti CD19 APC-R700 or anti-CD31 PE, anti-CD 184 APC (BD Biosciences-Pharmingen, San Diego, California, USA). The controls were isotype-matched mouse immunoglobulins. After each incubation performed at 4°C for 20 minutes, cells were washed in PBS 1X containing 1% heat-inactivated FCS and 0,1% sodium azide. The cytometric analyses were performed on a LYRIC flow cytometer using FACSuite™ software (BD Biosciences-Immunocytometry System). Each analysis included at least 1-2x 10⁴ events for each gate. A light-scatter gate was set up to eliminate cell debris from the analysis. The percentage of positive cells was assessed after correction for the percentage reactive to an isotype control conjugated to a specific fluorophore. Percentage of different cells subpopulations were calculated on the Syto™16 positive gate.

### Example 3 - In vitro evaluation of Angio^{PRP}

Separation of peripheral blood was performed through Regeneron device and the Angio^{PRP} was analysed to characterize the cell and platelet composition. As reported in figure 1A, Angio^{PRP} is composed mainly by platelets (85.19±7.03%), with a white blood cells (WBCs) presence of 0.85±0.53%. The platelets concentration is increased in Angio^{PRP} compared to the original number present in whole blood (146.8±11.35x10³ instead of 108.5±6.62x10³ platelets/µl, figure 1B). In particular, the cellular component was further characterized by a Coulter counter and compared with original peripheral blood composition, before the separation procedure. In Angio^{PRP} the lymphocyte component is enriched, compared to the whole blood (67.15±9.25% for Angio^{PRP} and 29.98±6.52% for whole blood), while the granulocyte population is severely reduced (12.32±7.67% for Angio^{PRP} and 62.36±7.38% for whole blood) and monocytes partially increased (20.13±6.30% for Angio^{PRP} and 7.69±1.56% for whole blood, figure ID.

### Example 4 - ex vivo preclinical experimentation: organotypic skin culture

A multilayered model of human dermis and epidermis (MatTek's EpiDermFT Full Thickness EFT-400) was used as in vitro model to better investigate the tissue regeneration capacity of Regeneron device product. Skin biopsies on tissue model (stratum corneum and epidermis) were obtained by 5mm punch as described by EpiDerm-FT manufacture's protocol. Four different culture conditions were tested: 1) an organotypic skin culture with Angio^{PRP}, 2) Angio^{cells}, 3) PRP, 4) PBS 1X (as negative control). We analyzed the human skin tissue after 24 hours, 2, 4, 5, 6 and 7 days of culture and we performed immunohistochemical and immunofluorescence analysis in order to evaluate wound closure and epithelium regeneration. Hematoxylin and Eosin staining was performed to better characterize wound healing; image quantification and process was performed with ImageJ software (NIH).

As reported in figure 2A, lesions treated with Angio^{PRP} showed a complete healing after 6 days, while the treatment with PRP took one more day to reach the same results The use of Angio^{cells} and PBS could not succeed the complete healing in one week.

### Example 5 - in vivo wound healing experiments

Five-month-old Scid mice (n=10) were obtained from Charles River Laboratories International, Inc. (Calco, Italy); the use of animals in this study was authorized by the National Ministry of Health (protocol number 51/2018-PR). Animals were anesthetized with avertin and two full-thickness excisions of 5-mm that include the panniculus carnosus are created on the dorsum, one on each side of the midline of the mouse. A silicone splint was placed around the wound with the assistance of adhesive and the splint was then secured with interrupted sutures. Each mouse acts as its own control, with one wound receiving treatment and the other phosphate buffer saline (PBS IX). A transparent occlusive dressing was applied to prevent contamination. Wounds were checked by taking photos every 2-3 days, and the area was quantified relative to a millimeter reference using ImageJ software (NIH) and expressed as the percentage of wound area measured at day 0, 4, 7 10, 15 and 21 days after injury, corresponding to wound closure; mice were sacrificed by cervical dislocation under full deep anesthesia and the back skin lesions were removed; the biopsies have been divided into two group respectively for histological or molecular biology analysis. One group was placed in isopentane and freezed at -80°C for proteomic analysis. The other group was incubated in 4% paraformaldehyde in PBS at 4°C overnight and after transferred to 30% sucrose in PBS 1X solution for a further 24 hour at 4°C, embedded in O.C.T compound and freezed at -80°C. Serial sections of 12 µm thickness were cut and examined by immunofluorescence and histological analysis.

We compared time of skin wound closure for Angio^{PRP} treatment (n=5), Hyalomatrix (n=5) and PBS treatment (n=10) respectively. Furthermore, we evaluated time required to reach a total closure in the different conditions. The results show that treatment with Angio^{PRP} at 21 days induces a complete closure of the lesion; instead, skin treated with Hyalomatrix showed a wound closure of 75-80% at 21 days after injury. Moreover, the samples treated with PBS showed 90% of wound closure at the same timepoint. The achievement of 60% of wound closure was observed after 6,5/7 days for Angio^{PRP} treatment, compared to 15 days for Hyalomatrix treatment (p<0,0001 one-way analysis of variance (ANOVA) with Bonferroni correction). The control treatment with PBS reaches 60% of closure at almost 10 days. A significant difference at 21 days was observed between Angio^{PRP} and Hyalomatrix (p<0,0001, two-way analysis of variance (ANOVA) with Bonferroni correction) and also between Angio^{PRP} and PBS (p<0,0001 two-way analysis of variance (ANOVA) Bonferroni correction) (Figure 2B).

### Example 6 - Epidermal differentiation

Serial section of 12 µm skin tissue sections were cut and stained with hemaetoxylin and eosin (H&E), Alcian Blue and Picrosirius Red staining according to the manufacturer's instructions for morphological assessment. Images were captured with LMD6000B (Leica, Germany).

For Immunofluorescence analysis tissue sections were incubated with mouse monoclonal antibody anti-Citokeratin 10 (1:100, Abcam), rabbit anti-Involucrin (1:100, abcam), mouse monoclonal antibody anti-Citokeratin 14 (1:100, Abcam) rabbit anti-loricrin (1:100, Abcam), CD206 (1:400, Abcam) Cell nuclei were stained for 5 min at room temperature with DAPI. The slides were analysed using a fluorescent microscope LEICA DMI8 (Leica, Germany).

After a period of 21 days hematoxylin and eosin staining reveals that the skin treated with Angio^{PRP}compared with the skin treated with Hyalomarix (commercial product) showed a complete wound healing induced by fibroblast and keratinocytes proliferation and migration to the wound site. We also evaluated the epidermal differentiation with specific marker for different epidermal layers. At 21 days, skin treated with Angio^{PRP} showed a complete and uniform regeneration of the basal layer as shown by positive and uniform signal for the specific marker Cytokeratin 5. On the contrary, the samples treated with Hyalomatrix and PBS showed an incomplete and discontinuous regeneration. Concerning granular layer, the treatment with Angio^{PRP} induced a complete regeneration, as demonstrated by Cytokeratin 10 staining, comparable with healthy skin sample. On the contrary, skin lesions treated with Hyalomatrix and PBS showed a late regeneration of the granular layers, with a non-homogeneous expression of cytokeratin 10. As regard stratum corneum, we performed an immunofluorescence staining for Loricrin in order to visualize the most external layer; we observed a complete re-epithelization only in samples treated with Angio^{PRP}, while in skin treated with Hyalomatrix and PBS we observed an incomplete epithelium at day 21.

### Example 7 - Skin elastic properties

Following sacrifice the skins for mechanical testing were placed in metal screw clamps with rubber pieces covering the clamped ends. Clamps were placed into a Bose Electroforce 3100 instrument. Applying an initial traction of 0.15 N. The traction measured in MPa was increased by 0.2% per second up to the breaking point. Force (N) and displacement (mm) were measured on a xy plotter and these points were subsequently recorded as stress (σ=force per cross-sectional area) and strain (ε=change in length/initial length) and re-plotted in Excel (Ashley W. Seifert et. Al; 2012).

To assess skin weakness, we compare mechanical properties of skin treated with Angio^{PRP}, PBS, Hyalomatrix (as negative control) and healthy skin (as positive control). We derived stress-strain curves from dorsal skin to determine the mean tensile strength. Skin treated with Angio^{PRP} showed a higher resistance to traction compared with skin treated with Hyalomatrix or with PBS (3MPa ± 0,7, 0,3MPa ± 0,3 and 1,8MPa ± 0,70) (Figure 2C). Moreover, skin treated with LPEP displayed a trend comparable with healthy skin (3MPa ± 0,7 and 3,1 ± 0,7). A higher modulus of elasticity observed in the skin treated with Angio^{PRP} can be due to an increased number of fibrillary molecules per unit area. Since elastin is the major constituent of skin elastic fibers and is beneficial for dermal regeneration, we performed an immunofluorescence staining for Collagen type VI. The skin treated with Angio^{PRP} showed a significant difference in terms of collage VI expression compared to the skin treated with Hyalomatrix, as quantified and reported in figure 2F (unpaired t-test, *p<0,05).

In addition to epidermal characterization, we performed an analysis of dermal composition. Alcian Blue staining was used to evidence the presence of collagen fibers in dermal area. Thanks to the intensity of this staining, it was possible to distinguish between high- and low-density elastic fibers in the skin samples and quantify the surface occupied by these two density types in the different treatments; as reported in figures 2D and 2E, samples treated with Angio^{PRP} show a dermal composition more similar to healthy skin then the Hyalomatrix and PBS conditions, with more high density elastin and less low density respectively (unpaired t-test, *p<0,05, **p<0,01, ****p<0,0001).

### Example 8 - proteomic and biochemical analysis

Skin samples were extracted for proteomic analysis, centrifuged at 17,000xg for 10 min at 4°C and supernatants were subjected to ultracentrifugation at 200,000xg for 1h at 4°C. Pellets, re-suspended in 0.1 M ammonium bicarbonate, pH 7.9, were trypsinized according procedures previously reported (Bari, Perteghella et al. 2018), using 50±0.5 µg of proteins from each sample. One microliter of trypsin-digested mixtures was analyzed by nano-cromatography equipped with cHiPLC-nanoflex system (Eksigent, AB SCIEX, USA) coupled to Q-Exactive mass spectrometer (Thermo Fisher Scientific, USA), through a 65 min gradient of 5-45% of eluent B (eluent A, 0.1% formic acid in water; eluent B, 0.1% formic acid in acetonitrile), at a flow-rate of 300 nL/min. Full mass spectra were recorded in positive ion mode over a 400-1600 m/z range at 70000 FWHM resolution, followed by 1 0 MS/MS spectra, at resolution of 17,500 FWHM, generated in data dependent manner on the most abundant ions. All data generated were searched using Proteome Discoverer 2.1 platform (Thermoscientific) based on SEQUEST search engine and human protein database (70726 entries, downloaded on January 2017 from UNIPROT website, www.uniprot.gov). The obtained protein lists were aligned, normalized (Sereni, Castiello et al. 2018) and then processed by means of Linear Discriminant Analysis (LDA) (Hilario and Kalousis 2008). For assigning each subject to specific group it was calculated the α-value parameter according the extracted marker proteins (Brambilla, Lavatelli et al. 2012).

In order to understand the protein profile of different wound treatments, we performed a nLC-MS/MS analysis; single PBS treatments were used as internal control for the relative group (Regeneron or Hyalomatrix), while healthy skin was analyzed to identify a normal control profile of the skin from the same animal (internal control). Total number of proteins identified was 1514, with a high technical and group reproducibility. Proteomic profiles from Hyalomatrix wound tissue are markedly different compared to profiles of control skin samples from the same animal. The principal component analysis of the samples shows that control samples form a similar cluster from samples collected from Angio^{PRP} treated wounds. In fact, samples treated with Hyalomatrix show a correspondence with their internal control, while single Angio^{PRP} samples correlates with their single relative internal control, suggesting a systemic effect of the treatment. Healthy skin samples clusterize and correlate more with Angio^{PRP} than Hyalomatrix group. After Bonferroni correction, 179 proteins were differentially expressed at least between 2 groups, although up- and down-regulated proteins were similar in the same comparison or condition evaluated. Analyzing the single treatment and its specific control, half number of differential proteins is changed for Angio^{PRP} then for Hyalomatrix treatment. The same behavior is reported if the treated conditions are compared to healthy skin. This suggest a trend for Angio^{PRP} closer to healthy skin than the Hyalomatrix one. The Proteome Recovery index (PRi) (Roffia, De Palma et al. 2018, Sereni, Castiello et al. 2018) was then applied to compare three different conditions, as the algorithm generates and compares healthy and disease profiles in order to identify the proteins involved in the homeostasis recovery. We identified 6 proteins that are absent in wounded tissue and recovered after Angio^{PRP} treatment, and 12 proteins present in the untreated lesion and not in Angio^{PRP} samples (PBS samples were used as negative controls). Levels of 97 proteins are recovered after Angio^{PRP} treatment (57 up-regulated and 40 down regulated in damaged tissue), while only 23 were detected after Hyalomatrix treatment (14 in common with Angio^{PRP}group). Clusterization of normalized data confirmed the results, with Hyalomatrix and relative controls in the same macro group, and healthy skin and Angio^{PRP} single samples correlating with their relative negative controls. The differentially expressed proteins were analyzed using Cytoscape platform to evaluate the metabolic. Network analysis underlined the variation of different metabolic clusters both for wound induction and two relative treatments. In particular, clusters relative to serpins, proteins involved in defense response, cytoskeleton organization, complement and coagulation cascades, extracellular matrix (ECM) and ribosome are overexpressed in the Hyalomatrix treatment and its control condition, while in Angio^{PRP} and relative control are normalized, as in healthy samples. On the opposite, clusters of keratins and proteins involved in lipid metabolism are more expressed in Angio^{PRP} conditions than in Hyalomatrix ones. Inside these eight target groups, we focused our attention on 17 wound-related proteins expressed in a different way after the two treatments and compared to the healthy tissue: Caveolin-1, EGFR, Fibronectin, Decorin, Plectin, SERPINB2, Discoidin domain receptor-2, Prothrombin, Heme Oxygenase 1, Fibrinogen alpha- beta- and gamma- chains, Tenascin, Plastin-2, Alpha-Tropomyosin, Cytokeratin-6A and Annexin A1. The expression of these proteins displays an opposite trend for Hyalomatrix and Angio^{PRP} treatments: for example, Caveolin 1 and Decorin are up regulated after Angio^{PRP} treatment, but down-regulated in Hyalomatrix samples. On the opposite, factors involved in coagulation, such as fibrinogen, SERPINB2 and Prothrombin are more expressed after Hyalomatrix then Angio^{PRP} treatment.

### Example 9 - network and statistical analysis

Starting from the list of proteins selected by differentially and LDA procedures, the corresponding *Homo sapiens* Protein-Protein Interaction (PPI) network was extracted, and by means of Cytoscape plug-in, STRING 8 database (Pawlowski, Muszewska et al. 2010), known interactions were retrieved from several databases such as Prolink, DIP, KEGG and BIND. In addition, PPI were examined using Cytoscape 3.5 (Shannon, Markiel et al. 2003) and only experimentally verified interaction with >0.15 score were retained. Finally, Bingo 2.44 (Maere, Heymans et al. 2005) was used to emphasize modules based on functionally organized GO terms.

Criteria for identification of peptide sequences and related proteins were: trypsin as enzyme; three missed cleavages permitted per peptide; mass tolerances of 10 ppm for precursor ions and ±0.6 Da for fragment ions were applied. Percolator node was used with target-decoy strategy to give final false discovery rates (FDR) at Peptide Spectrum Match (PSM) level of 0.01 (strict) based on q-values, considering maximum deltaCN of 0.05 (Kall, Canterbury et al. 2007); minimum peptide length of six amino acids, and rank 1 were considered, and protein grouping and strict parsimony principle were applied.

Differences of categorical variables were evaluated using X-squared test. Kruskal Wallis test was applied for comparing quantitative variables. Analyses were performed using R (3.5.2) statistical analysis software; p-value <0.05 was considered statistically significant.

SpCs were evaluated by t-test, too (Zhang, VerBerkmoes et al. 2006), proteins selected by both LDA and MAProMA were evaluated by hierarchical clustering (Zhao and Karypis 2005) applying Ward's method and Euclidean distance metric. LDA. using common covariance matrix for all stratified groups and Mahalanobis distance (Jain AK 1999) and hierarchical clustering were performed by JMP 5.1 software. To select proteins discriminating the stratified children groups, we considered those with largest F ratio (≥5) and smallest p-value (≤0.05). Based on a direct correlation between the spectral counts (SpC, also called PSM) and relative abundance of identified proteins, DAve (Differential Average) and DCI (Differential Coefficient Index) indices of MAProMa (Multidimensional Algorithm Protein Map) software (Mauri, Riccio et al. 2014) were used to process average (aSpC) corresponding to each analyzed children group. The threshold values imposed were DAve >|0.2| and DCI>|001|.

## Claims

1. An isolated blood preparation consisting of platelet- and lymphocyte-enriched plasma, comprising:
- platelets at a concentration ranging from 110 x 10⁶/ml to 330 x 10⁶/ml, preferably from 150x10⁶/ml to 330 x 10⁶/ml;
- white blood cells CD45⁺, CD31⁺ and CD34⁻ at a concentration ranging from 0.1x10⁶/ml to 2.0x10⁶/ml, preferably from 0.1x10⁶/ml to 0.7x10⁶/ml;
- red blood cells at a concentration ranging from 0 to 0.03x10⁹/ml, preferably less than 0.01x10⁹/ml,
for use in wound healing and/or in the regeneration of skin tissues.

2. The isolated blood preparation for use according to claim 1, wherein the white blood cells CD45⁺, CD31⁺ and CD34⁻ comprise lymphocytes, monocytes and granulocytes.

3. The isolated blood preparation for use according to claim 2, wherein lymphocytes range from 46.2% to 80.6%, preferably from 61.6% to 73.7% of total white blood cells CD45⁺, CD31⁺ and CD34⁻.

4. The isolated blood preparation for use according to claims 2-3, wherein lymphocytes comprise T cells, angiogenic T lymphocytes (Tang), B cells and NK cells.

5. The isolated blood preparation for use according to claim 2, wherein monocytes are CD14⁺ and CD16⁺ or CD16⁻.

6. The isolated blood preparation for use according to claims 2 and 5, wherein said monocytes range from 7.7% to 40.0%, preferably from 15.9% to 23.1%, of total white blood cells CD45⁺, CD31⁺ and CD34⁻.

7. The isolated blood preparation for use according to claim 2, wherein granulocytes are CD15⁺/16⁺.

8. The isolated blood preparation for use according to claims 2 and 7, wherein granulocytes range from 3.3% to 32.9%, preferably from 5.9% to 16.7%, of total white blood cells CD45⁺, CD31⁺ and CD34⁻.

9. A method for preparing the blood preparation of claims 1-8, which comprises the following steps:
(i) centrifuging from 2 to 7ml of whole blood in a centrifugation tube at 1200-1800 rpm, preferably at 1500 rpm (rounds per minute), for 5 up to 10 min at room temperature, thereby obtaining the separation of an upper and lower phase, wherein the upper phase contains plasma enriched in platelets and lymphocytes and the lower phase contains red cells;
(ii) collecting the upper phase containing the plasma enriched in platelets and lymphocytes.

10. The method of claim 9, wherein in step (i) from 2 to 4ml of whole blood are centrifuged at 1500rpm for 5 min at room temperature.

11. The method of claims 9-10, wherein the centrifugation tube is provided with a sept separating the tube in two parts so as to enable the physical separation of the platelet- and lymphocyte-rich plasma phase from red cells.

12. The method of claim 11, wherein the sept consists of a hydrophobic disk barrier or membrane with less than 200 micron porosity and provided with a central hole of 1.5 to 2.5 mm diameter.

13. A blood preparation obtainable by the method of claims 9-12.

14. The blood preparation according to claims 1-8 or 13, for use in the treatment of lesions of orthopedic or oncological origin such as bone and cartilage damages, post-surgery lesions or wounds; skin ulcers, decubitus sores, pressure ulcers, venous leg ulcers, arterial ulcers, diabetic foot ulcers, corneal lesions such as scarring of the cornea and corneal ulcers; periodontal tissue damages or periosteal lesions.

15. The use of isolated blood preparation consisting of platelet- and lymphocyte-enriched plasma as defined in claims 1-8 or 13 for the following cosmetic treatments: anti-aging, scar repairing, wrinkle, acne, alopecia and burns.

16. A pharmaceutical or cosmetic composition containing the isolated blood preparation of claims 1-8 or 13.
